# EUROPÄISCHE PATENTSCHRIFT

(11) **EP 2 169 221 B1**
(45) Veröffentlichungstag und Bekanntmachung des Hinweises auf die Patenterteilung: **15.07.2015**
(21) Anmeldenummer: 09011978.5
(22) Anmeldetag: 21.09.2009
(51) Int. Cl.: F03D 7/04, F03D 11/00, G01N 33/28, F03D 11/02, F16H 57/04

(54) **Verfahren zum Überwachen eines Getriebes einer Windenergieanlage**
Method for monitoring a transmission system of a wind energy assembly
Procédé de surveillance d'un engrenage d'éolienne

(30) Priorität: 25.09.2008 DE 102008048956
(43) Veröffentlichungstag der Anmeldung: 31.03.2010
(73) Patentinhaber: Senvion SE, 22297 Hamburg (DE)
(72) Erfinder: Ehlers, Marco, 24811 Owschlag (DE)
(74) Vertreter: Seemann, Ralph

(56) Entgegenhaltungen:
- EP-A1- 1 795 892
- EP-A2- 0 159 094
- WO-A1-2007/088015
- DE-A1- 10 230 759
- DE-B3- 10 343 457
- US-A- 5 760 298

## Beschreibung

Die Erfindung betrifft ein Verfahren zum Überwachen eines Getriebes einer Windenergieanlage mit einem Getriebeflüssigkeitskreislauf, ein Computerprogramm sowie ein Überwachungssystem für ein Getriebe einer Windenergieanlage mit einem Getriebeflüssigkeitskreislauf.

In vielen modernen Windenergieanlagen wird die windgetriebene Rotation des Rotors der Windenergieanlage über ein Getriebe an einen Generator weitergeleitet. Dabei wirken auf das Getriebe der Windenergieanlage starke Kräfte, die zu Verschleiß und Abrieb an beweglichen Bauteilen des Getriebes führen. Unter anderem die bei extremen Windböen auf das Getriebe einwirkenden extremen Kräfte können auch zu schwerwiegenden Getriebeschäden führen. Für einen einwandfreien und sicheren Betrieb der Windenergieanlage ist es somit notwendig zu überwachen, dass das Getriebe in einem ordnungsgemäßen und betriebsbereiten Zustand ist und der Verschleiß nicht zu Schäden im Getriebe führt, die sicherheitsrelevant sind oder zu unnötigen und unwirtschaftlichen Stillstandszeiten bei der Windenergieanlage führen.

Zur Getriebediagnose bei Windenergieanlagen werden in der Regel aufwendige Diagnosesysteme benutzt, die über Körperschallsensoren, Mikrofone oder Beschleunigungssensoren die Schwingungsfrequenzen des Getriebes überwachen. Veränderungen der Frequenzspektren deuten auf Getriebeschäden hin. Wesentlich für solche Diagnosesysteme ist eine hohe Fehlererkennungsquote, vorzugsweise größer als 99%, bei möglichst geringer Quote von Fehlauslösungen. Bei bekannten Diagnosesystemen ist daher eine Begutachtung der aufgezeichneten Frequenzspektren durch einen Experten erforderlich, da die Zusammenhänge bei der Entstehung von Frequenzspektren von Getrieben zu komplex sind, als dass sie von einem vollautomatischen System so ausgewertet werden könnten, dass Fehler sicher erkannt werden, ohne dass es zu Fehlauslösungen kommt. Auch angesichts der unterschiedlichen Getriebetypen, die in modernen kommerziellen Windenergieaniagen zum Einsatz kommen und die unterschiedliche Verhalten und Frequenzspektren aufweisen, werden derzeit keine vollautomatischen Diagnosesysteme eingesetzt.

In der Praxis werden bei den bekannten Diagnosesystemen Grenzwerte für Abweichungen von Soliwerten so niedrig eingestellt, dass Fehler sicher erkannt werden. Dies führt allerdings auch zu einer hohen Zahl von Fehlauslösungen. Zur Vermeidung von unnötigen Stillstandszeiten wird eine Grenzwertüberschreitung daher nur mit einer Alarmmeldung quittiert. Eine Veränderung der Betriebsführung in Reaktion auf einen Getriebefehler erfolgt erst nach der Begutachtung der aufgezeichneten Daten durch eine fachkundige Bedienperson.

Beispielsweise führen extreme Wetterlagen bei Windparks mit mehreren Windenergieanlagen zu einer Vielzahl von Alarmmeldungen, die in einer Überwachungszentrale zusammenlaufen. Da allen Alarmmeldungen durch die Bedienpersonen im Überwachungszentrum nachgegangen werden muss, um festzustellen, ob es sich um Fehlalarme oder tatsächliche Fehler handelt, ist es möglich, dass in einer solchen Situation tatsächliche Fehler nicht rechtzeitig erkannt werden. Dies kann zu unnötigen Schäden und Anlagenausfällen führen.

DE 103 43 457 B3 offenbart eine Vorrichtung zur Partikelmessung in einem Fluidstrom eines viskosen Mediums, bei dem es sich insbesondere um Getriebeöl handelt. Mithilfe einer Einstelleinrichtung wird der Eingangsdruck des viskosen Mediums vor Eintritt des Fluidstroms in die Sensormesszone des Partikelzählers angepasst. Dies dient der Anpassung des Eingangsdrucks an die temperaturabhängige Viskosität des Mediums, beispielsweise des Hydrauliköls.

WO-A-2007/088015 zeigt eine Vorrichtung zur Detektion von Partikeln in einem Fluidstrom. Die Vorrichtung umfasst eine Feldspule zur Erzeugung eines Magnetfelds und zwei gegensinnig gewickelte Sensorspulen, die mit einer Auswerteeinrichtung verbindbar ist. Anhand eines in den Sensorspulen induzierten Signals wird die Anwesenheit von Partikeln in dem Fluidstrom detektiert. Die Vorrichtung ist sowohl zur Überwachung eines Gesamtsystems als auch für die Verschleißüberwachung einzelner Komponenten einsetzbar. Ausgehend von der Partikelbelastung des Fluidstroms werden unmittelbare Rückschlüsse auf den Zustand der Komponenten gezogen.

EP-A-1 795 892 zeigt einen Detektor zur Überwachung eines Schmiermittels einer Vorrichtung, welcher eine Mantelelektrode umfasst, in deren Innenbereich ein Stabmagnet vorhanden ist. Dieser dient dazu, ferromagnetische Partikel anzuziehen. Eine elektronische Schaltung detektiert, wenn die Anzahl der angelagerten Teilchen so groß ist, dass die Elektroden in Kontakt miteinander kommen, und gibt eine Fehlermeldung aus. Mit diesem Detektor ist der Verschleiß mechanischer Komponenten, insbesondere einer Windkraftanlage, überwachbar, wobei ein Alarm ausgegeben wird, bevor ernsthafte Fehlfunktionen auftreten.

DE 102 30 759 A1 betrifft ein Verfahren zur Überwachung und Diagnose einer Maschine unter Verwendung eines Messsystems mit einer auf einen Spulenkern gewickelten Spule, auf deren Oberfläche sich die zu detektierenden ferritischen Abriebpartikeln anlagern. Ein dem Spulenkern gegenüberliegendes rotierendes Zahnrad beeinflusst die Induktivität der Spule. Das Ausgangssignal der Spule weist Impulse mit gleichbleibender Amplitude auf, deren Frequenz von der Rotationsgeschwindigkeit des Zahnrades abhängt. Abweichungen in der Amplitudenhöhe sind auf die Anlagerung von ferritischen Abriebpartikeln an der Spule zurückführen und daher ein Maß für den Zustand der überwachten Maschine bzw. des überwachten Getriebes.

EP-A-0 159 094 offenbart eine Vorrichtung zur Erfassung von metallischen Verunreinigungspartikeln in einem Fluidstrom, beispielsweise einem Schmierölsystem einer Kolbenmaschine oder eines Getriebes. Die Detektionsvorrichtung umfasst ein Gitter mit verschieden großen Öffnungen, an welchen jeweils elektrische Kontakte vorhanden sind. Verfängt sich ein Metallpartikel in einer solchen Öffnung, wird ein Kontakt geschlossen. Eine außerdem vorgesehe ne Schaltung sorgt dafür, dass eine überwachte Maschine abschaltet oder ein Alarm ausgelöst wird.

US 5,760,298 zeigt ein System zur Detektion von Ablagerungen in einem Fluid durch Überwachung von dessen elektrostatischen Eigenschaften. Dieses Überwachungssystem ist insbesondere für Öl und überhitzten Dampf einsetzbar. Das System ist in der Lage, stetig zunehmende Ablagerungen oder plötzliche auftretende und starke Steigerungen des Kontaminationsgrades festzustellen. Als Reaktion kann das System einen Alarm erzeugen, beispielsweise wenn das Verschmutzungsniveau einen vorbestimmten Schwellwert erreicht oder sich plötzlich stark erhöht.

Gegenüber diesem Stand der Technik liegt der Erfindung die Aufgabe zugrunde, die Überwachung eines Getriebes einer Windenergieanlage zu vereinfachen, zu automatisieren und deren Zuverlässigkeit zu verbessern.

Diese Aufgabe wird gelöst durch ein Verfahren zum Überwachen eines Getriebes einer Windenergieanlage gemäß Anspruch 1.

Als Getriebeflüssigkeit wird im Rahmen der vorliegenden Erfindung ein Schmiermittel, etwa ein Schmieröl oder Getriebeöl, bezeichnet, das in einem Flüssigkeitskreislauf zur Schmierung eines Getriebes einer Windenergieanlage eingesetzt wird.

Die Erfindung beruht auf dem Gedanken, dass in einer Getriebeflüssigkeit durch Reibung der beweglichen Getriebekomponenten aneinander Partikel abgerieben werden und dabei in den Getriebeflüssigkeitskreislauf gelangen. Die Messung der Anzahl, Größe und/oder Art dieser Partikel erlaubt Rückschlüsse auf den Zustand des Getriebes.

Im Vergleich zu herkömmlichen Methoden bietet die Messung der Anzahlen, Größen und/oder Arten von Partikeln, die aus dem Verschleiß des Getriebes resultieren, eine sehr einfache Messmethode, die automatisierbar ist. Aufgrund der vergleichsweise geringen Datenmengen ist auch kein Expertenwissen notwendig. Die Messung ist so wenig komplex, dass sie auch bei automatischer Auswertung wenig fehleranfällig ist.

Bereits diese vergleichsweise einfache Messung erlaubt überraschenderweise präzise und verwertbare Voraussagen Ober das Vorliegen eines erhöhten Verschleißes und einer drohenden Überlastung des Getriebes bzw. Ober das Vorliegen eines Getriebeschadens. Zwar ist nicht in jedem Fall eine Veränderung von Betriebsparametern der Windenergieanlage notwendig, so dass es in vielen Fällen ausreicht, eine entsprechende Statusmitteilung zu erzeugen. Aufgrund der Einfachheit der Messung kann auch eine solche Statusmitteilung zuverlässig Aufschluss über den Zustand des Getriebes der Windenergieanlage geben. Darüber hinaus können beim Auftreten erhöhter Messwerte jedoch gegebenenfalls automatisch Gegenmaßnahmen ergriffen werden, um die Belastung des Getriebes zu verringern.

Das erfindungsgemäße Verfahren spart Kosten, da unnötige Stillstandszeiten vermieden werden, der technische Geräteaufwand erheblich reduziert ist und kein teures fachkundiges Überwachungspersonal zur Begutachtung der Messergebnisse erforderlich ist.

Erfindungsgemäß ist es möglich, einen Grenzwert zu verwenden, oder mehrere Grenzwerte. Diese werden dann im Rahmen dieser Erfindung als erster, zweiter usw. Grenzwert bezeichnet.

Erfindungsgemäß ist vorgesehen, dass bei Überschreiten wenigstens eines ersten Grenzwertes durch einen Messwert eine Statusmitteilung erzeugt wird und bei Überschreiten wenigstens eines zweiten Grenzwertes durch einen Messwert ein Betriebsparameter der Windenergieanlage verändert wird. Es können dabei eine oder mehrere Alarmstufen bzw. Statusmitteilungen vor der Veränderung eines Betriebsparameters der Windenergieanlage vorgesehen sein. Auch das Verändern eines Betriebsparameters der Windenergieanlage geht vorteilhafterweise mit einer Statusmittlung über die Veränderung einher. So ist es einer Bedienperson in einem Fernüberwachungszentrum möglich, in informierter Weise in den automatischen Überwachungs- und Steuerungsvorgang der Windenergieanlage einzugreifen. Die Statusmitteiiung gibt der Bedienperson dabei die notwendigen Informationen über den Grund der Statusmitteilung und/oder für die automatische Änderung der Betriebsparameter.

Erfindungsgemäß wird zur Messung der Anzahl und/oder Größe und/oder Art der in der Getriebeflüssigkeit enthaltenen Partikel mittels wenigstens eines Partikelzählers die Größe und/oder Anzahl und/oder Art von durch einen Getriebeflüssigkeitsleitungsquerschnitt strömenden Partikeln gemessen. Dabei kommen beispielsweise optische und/oder induktive Partikelzähler zum Einsatz. Bevorzugt werden metallische Partikel gemessen, insbesondere mittels wenigstens eines induktiven Partikelzählers.

Ein Beispiel eines bekannten induktiven Partikelzählers ist in WO 2007/088015 offenbart. Mittels solcher induktiver Partikelzähler ist feststellbar, ob es sich um metallische bzw. magnetisierbare Partikel handelt oder nicht. Mittels der Höhe des Signals des induktiven Partikelzählers können Rückschlüsse auf die Größe der gemessenen Partikel gezogen werden. Durch Kopplung von optischen und induktiven Partikelzählern sind weitere Diskriminierungen zwischen nicht-metallischen und metallischen Partikeln und Partikeln verschiedener Metalle oder verschiedener Nicht-Metalle möglich. Da in Getrieben von Windenergieanlagen hauptsächlich metallische Werkstoffe verarbeitet werden, sind induktive Partikelzähler besonders bevorzugt.

Vorteilhafterweise findet die Messung im Getriebeflüssigkeitskreislauf stromaufwärts eines Partikelfilters statt. Somit werden die Partikel im Getriebeflüssigkeitskreislauf gemessen, bevor sie durch einen Partikelfilter, beispielsweise einen Ölfilter, aus dem Kreislauf entfernt werden. Die ausgefilterten Partikel verursachen keine weiteren Schäden. Die Messungen der Partikeizahlen sind aktuell und nicht kumulativ, da sich die Partikel in der Getriebeflüssigkeit nicht anreichern.

Die Veränderung von Betriebsparametern betrifft vorzugsweise einen Blatteinstellwinkel und/oder ein Generatormoment. Die Windenergieanlage kann auch still gesetzt werden, was durch Veränderung des Blatteinstellwinkels und/oder des Generatormoments geschehen kann. Vorzugsweise führen Veränderungen der Betriebsparameter zu einer Entlastung des Getriebes.

Vorzugsweise ist vorgesehen, dass wenigstens ein Grenzwert eine kumulierte Anzahl von Partikeln, eine Anzahl von Partikeln pro Zeiteinheit, eine Veränderung der Anzahl von Partikeln pro Zeiteinheit, eine absolute Größe von Partikeln, eine Größenverteilung von Partikeln, eine Veränderung der Partikeigrößenverteilung, die Art der Partikel oder eine Verteilung verschiedener Partikelarten oder eine Veränderung der Verteilung von Partikelarten oder ein aus verschiedenen Messwerten gebildeter abgeleiteter Wert ist. Dabei wird unter einer kumulierten Anzahl von Partikeln insbesondere die Gesamtzahl der seit Inbetriebnahme eines Getriebes gemessenen Partikel verstanden. Insbesondere ist der Grenzwert festlegbar auf eine Anzahl von 10 bis 500, vorzugsweise 25 bis 50, insbesondere vorzugsweise 25 bis 30, Partikeln in 10 Minuten, auf eine Anzahl von 50 bis 20.000, insbesondere 500 bis 1.000, Partikeln in 24 Stunden oder auf eine kumulierte Anzahl von 200.000 bis 1.000.000, insbesondere 300.000 bis 400.000, Partikeln. Die Zeiteinheiten sind vorzugsweise gleitende Zeiträume, so dass beispielsweise jederzeit feststellbar ist, wie viele Partikel in den letzten 10 Minuten gemessen wurden. Alternativ vorzugsweise für eine einfachere Analyse bzw. Messung sind die Zeiträume aufeinander folgender Zeitabschnitte, etwa aufeinander folgende 10-Minutenabschnitte oder aufeinander folgende Tage.

Die Messung der Veränderung der Verteilung der Partikelarten und Partikelgrößen erfordert eine längere Zeit als die Messung von absoluten oder kumulierten Partikelzahlen bzw. Partikelnzahlen pro Zeiteinheit. Zur Schnellerkennung von Getriebeschäden werden bevorzugt die schneller ermittelbaren Messdaten verwendet. Neben den schneller ermittelbaren Messdaten werden die zeitlich aufwendigeren Messwerte überwiegend für die längerfristige Statusdiagnose des Getriebes verwendet. Diese verschiedenen Messwerte geben eine umfangreiche Datenbasis zur Beurteilung, auch zur automatischen Beurteilung, von bestimmten Verschleißerscheinungen an.

Nach einem Komponententausch oder einer Wartungs- u. o. Reparaturmaßnahme sind ggf. die Grenzwerte ggf. anzupassen bzw. die Partikelzählschleifen zu initialisieren, z.B. muss der Absolutwertzähler nach einem Getriebetausch auf Null gesetzt werden u. der Grenzwert pro Zeiteinheit wieder auf den Wert für die Einlaufphase gesetzt werden. Ebenso können nach einem Nachschleifen einer Verzahnung für einen begrenzten Zeitraum deutlich höhere Grenzwerte erforderlich sein.

In einer vorteilhaften Weiterentwicklung von eigenständigem erfinderischem Rang ist vorgesehen, dass wenigstens ein Grenzwert abhängig vom Betriebszyklus der Windenergieanlage verändert wird, insbesondere abhängig vom Betriebszustand, insbesondere Stillstand, Trudel-, Teillast- und/oder Volllastbetrieb des Getriebes und/oder abhängig vom Alter des Getriebes, insbesondere anhand des Lebens- oder Verschleißalters, der Betriebsstunden und/oder der Volllaststunden des Getriebes.

Im Rahmen der vorliegenden Erfindung wird unter einem Betriebeszyklus sowohl der Betriebszustand, d. h. Stillstand, Trudelbetrieb, Teillastbetrieb bzw. Volllastbetrieb, als auch das Lebensalter bzw. Verschleißalter des Getriebes anhand des absoluten Lebensalters des Getriebes, der Betriebsstunden des Getriebes bzw. der Volllaststunden des Getriebes verstanden.

Es ist vorgesehen, dass bei Stillstand der Windenergieanlage andere Grenzwerte gelten als im Trudelbetrieb, bei Teillast und bei Volllast. Insbesondere werden Grenzwerte für wenigstens einen Messwert von Stillstand über Trudelbetrieb, Teillastbetrieb bis hin zu Volllastbetreib schrittweise oder kontinuierlich erhöht.

In Bezug auf das Lebensalter bzw. die Betriebsstunden oder Volllaststunden des Getriebes ist zu berücksichtigen, dass ein neues Getriebe Komponenten aufweist, die noch nicht aufeinander eingefahren bzw. eingeschliffen sind. In der sogenannten Einlaufphase zu Beginn des Einsatzes eines neuen Getriebes setzt daher ein vergleichsweise hoher Verschleiß ein, der daraus resultiert, dass die Komponenten sich aneinander reiben und überstehende Grate und ähnliche Artefakte der Produktion, die dem reibungsarmen Betrieb des Getriebes entgegenstehen, abgeschliffen werden. Die resultierende hohe Zahl von Partikeln in der Getriebeflüssigkeit ist daher kein Zeichen für Getriebeschäden. Deshalb ist in der Einlaufphase vorzugsweise eine hohe Zahl von Partikeln in der Getriebeflüssigkeit zu tolerieren.

Nachdem die Getriebekomponenten sich aufeinander eingestellt und eingeschliffen haben, geht die Anzahl der Partikel in der Getriebeflüssigkeit zurück. Daher wird vorzugsweise wenigstens ein Grenzwert während einer Einlaufphase höher gesetzt als nach der Einlaufphase. Nach der Einlaufphase des Getriebes weisen ähnlich hohe Anzahlen von Partikeln in der Getriebefiüssigkeit auf einen Getriebeschaden hin. Da mit dem Alter des Getriebes der Verschleiß zunimmt, wird nach der Einlaufphase der wenigstens eine herabgesetzte Grenzwert mit zunehmender Laufzeit des Getriebes erhöht.

Vorzugsweise wird wenigstens ein Grenzwert für einen Messwert und/oder der zeitliche Verlauf des wenigstens einen Grenzwertes durch eine statistische Auswertung von Messwertdaten mehrerer Windenergieanlagen mit gleichen oder ähnlichen Getriebetypen in Abhängigkeit vom Betriebszyklus bestimmt, wobei insbesondere die Streuung der Messwerte bei der Bestimmung des Grenzwertes einfließt. Auf diese Weise entsteht eine umfangreiche Datenbasis von vergleichbaren Messwerten, anhand derer Grenzwerte für einen einzelnen oder für verschiedene Messwerte mit statistischen Methoden bestimmt werden können. Jedes Getriebe ist ein Einzelstück und unterscheidet sich innerhalb von Fertigungstoleranzen von anderen Getrieben desselben Typs. Da außerdem verschiedene Windenergieanlagen niemals unter exakt gleichen Bedingungen betrieben werden, ergibt sich eine gewisse Streubreite bei den Messwertdaten. Die statistische Auswertung insbesondere der Streuung der Messwerte erlaubt daher Rückschlüsse auf die Wahrscheinlichkeit, dass ein Messwert einem tatsächlichen Schaden entspricht. Vorzugsweise entspricht der wenigstens eine in der statistischen Auswertung bestimmte Grenzwert einer mindestens 95%igen, insbesondere einer 99%igen, Fehlererkennungsquote.

Die vorliegenden umfangreichen Daten reflektieren die Alterung der Getriebe in Abhängigkeit der absoluten Zeit, der Betriebsstunden bzw. Volllaststunden des Getriebes ebenso wie den Stillstand, den Trudelbetrieb, den Teillastbetrieb und den Volllastbetrieb im Verlauf des Getriebelebens. So fallen bei jeder Windenergieanlage im Jahr über 50.000 Datensätze für Messungen in 10-Minuten-Intervallen an. Wenn beispielsweise die Daten von 20 Windenergieanlagen zusammengenommen werden, sind bereits für ein Jahr mehr als eine Million Messdatensätze vorhanden, die verschiedene Betriebszustände und Wetterbedingungen wiedergeben.

Daher können die umfangreichen Messdatenverteilungen in Abhängigkeit des Betriebszyklus, also abhängig von Betriebsart und Lebensalter des Getriebes, mit statistischen Methoden ausgewertet werden, um Grenzwerte zu bestimmen, bei deren Überschreiten mit 99%iger Sicherheit ein vorliegender Getriebeschaden erkannt wird. Ebenso lässt sich mit statistischen Methoden aus diesem Datenbestand schlussfolgern, wie dringend eine instandsetzungsmaßnahme an einem Getriebe ausgeführt werden muss und dementsprechend ein Termin zur Durchführung einer instandsetzungsmaßnahme vorschlagen.

Die Messdatenverteilungen weisen im Allgemeinen eine statistische Verteilung auf, etwa eine Normalverteilung oder eine X²-Verteilung (wobei X der griechische Buchstabe "Chi" ist). Bei einer Normalverteilung wird eine Fehlererkennungsquote von 95% oder 99% beispielsweise erreicht, indem ein Grenzwert um drei bzw. vier Standardabweichungen oberhalb des Mittelwerts für einen bestimmten Messwert angesetzt wird. Entsprechende Kriterien sind auch für andere Verteilungstypen bekannt. Ebenso wird auch die Fehlauslösungsquote minimiert.

Im Einzelfall weist ein Getriebe im Durchschnitt mehr oder weniger Verschleiß oder andersartigen Verschleiß auf als der Durchschnitt der Getriebe in der Messdatensammlung, anhand derer die Grenzwerte bestimmt werden. Dies drückt sich beispielsweise darin aus, dass für das Getriebe einer Windenergieanlage im Durchschnitt des Regelbetriebs der Windenergieanlage eine von dem Durchschnitt der Referenzmessdaten abweichende Anzahl, Größenverteilung oder Artenverteilung von Partikeln in der Getriebeflüssigkeit gemessen wird. Dies kann dazu führen, dass die statistische Auswertung der Referenzdaten für das einzelne Getriebe korrigiert werden muss.

Aus diesem Grund ist vorteilhafterweise vorgesehen, dass für ein Getriebe wenigstens ein Grenzwert anhand der Messwerte und/oder des bisherigen zeitlichen Verlaufs der Messwerte des Getriebes korrigiert wird. Auf diese Weise werden Besonderheiten des einzelnen Getriebes bei der Überwachung des Getriebes berücksichtigt. So ist es beispielsweise möglich, bei einem Getriebe, das bekanntermaßen eine überdurchschnittliche Anzahl von Partikeln aufweist, bei dem jedoch in Inspektionen oder bei instandhaltungsmaßnahmen keine erhöhte Ausfallgefahr diagnostiziert worden ist, den Grenzwert für die Anzahl der Partikel pro Zeiteinheit heraufzusetzen, um keine unnötigen Alarme zu erzeugen. Hierbei muss gewährleistet sein, dass eine solche Erhöhung nicht auf Kosten der Betriebssicherheit geschieht.

Vorzugsweise werden Getriebe, bei denen Getriebeschäden aufgetreten sind, bei der statistischen Auswertung zur Ermittlung eines Grenzwertes oder des zeitlichen Verlaufs eines Grenzwertes, der insbesondere einem sicheren Betrieb des Getriebes entspricht, nicht berücksichtigt. Diese werden vorzugsweise zur Erkennung von Getriebeschäden berücksichtigt, insbesondere anhand der für bestimmte Getriebeschäden charakteristischen zeitlichen Verläufe eines oder mehrerer Messwerte. So ist vorteilhafterweise bei bekannten Vorfällen, bei denen ein Getriebe Schaden genommen hat, anhand der genommenen Messwerte von Partikeln in der Getriebeflüssigkeit eine Korrelation zwischen den Messwerten und der Art des. Getriebeschadens möglich, die in die Überwachung eines Getriebes einfließen. Dementsprechend können zielgerichteter und flexibler als bisher Statusmitteilungen über vorliegende oder drohende Getriebeschäden mitgeteilt werden und gegebenenfalls Veränderungen in Betriebsparametern vorgenommen werden.

Dementsprechend ist vorzugsweise vorgesehen, dass aus der Analyse des zu messenden zeitlichen Verlaufs wenigstens eines Messwertes, insbesondere durch Vergleich des Messwertes mit Messwerten von anderen Windenergieanlagen mit gleichen oder ähnlichen Getriebetypen, ein Schadensfall erkannt wird und eine entsprechende Statusmitteilung generiert wird und/oder Betriebsparameter der Windenergieanlage geändert werden.

Weiterhin ist vorteilhaft vorgesehen, dass die in einer Datenbank gesammelten zeitlichen Verläufe der Messgrößen von Getrieben, bei denen ein Schaden aufgetreten ist, genutzt werden, um einen neuen Überwachungsalgorithmus oder neue Grenzwertdefinitionen zu validieren. Erkennt der Überwachungsalgorithmus den aufkommenden (historischen) Schaden anhand der gespeicherten Daten nicht rechtzeitig, ist er nicht für eine Überwachung geeignet und muss modifiziert werden. Auf diese Weise lassen sich halbempirisch sehr zuverlässige Algorithmen entwerfen.

Vorzugsweise wird aus der Analyse des gemessenen zeitlichen Verlaufs wenigstens eines Messwertes, insbesondere durch Vergleich des Messwertes mit Messwerten von anderen Windenergieanlagen mit gleichen oder ähnlichen Getriebetypen, die zu erwartende Restlaufzeit des Getriebes und/oder ein Termin für eine instandhaltungsmaßnahme für das Getriebe ermittelt. Erfindungsgemäß wird dabei unter einer instandhaltungsmaßnahme insbesondere eine Wartung des Getriebes, eine Überprüfung des Getriebes mit oder ohne Ausbau von Getriebebauteilen, ein Wechsel der Getriebeflüssigkeit, ein Austausch oder eine Reparatur von Messgeräten, insbesondere eines Partikelzählers, eine Reparatur des Getriebes vor Ort oder im teilweise oder ganz ausgebauten Zustand und/oder der Austausch eines Getriebebauteils oder des Getriebes verstanden.

Für eine zusätzliche Vereinfachung sorgt die vorteilhafte Weiterentwicklung, dass mehrere Messwerte mit verschiedenen Gewichtungen, insbesondere für verschiedene Partikelgrößen, ausgewertet werden, wobei die Gewichtungen der Messwerte ihrer indikatoreigenschaft für Alarmzustände und/oder Schadenszustände eines Getriebes entsprechen. So treten sicherheitsrelevante Getriebeschäden häufig zusammen mit einer erhöhten Anzahl großer Partikel auf, die auf Verschleißerscheinungen zurückgehen, die strukturelle Integrität des Getriebes beeinträchtigen, während Häufungen von kleineren Partikeln weniger sicherheitsrelevant sein können. Somit können für Statusmitteilungen oder Betriebsänderungen in Sachen Betriebssicherheit größere Partikel vorteilhafterweise stärker gewichtet werden als kleinere Partikel.

Insbesondere hat es sich als vorteilhaft erwiesen, wenn anstelle oder ergänzend zur Verwendung absoluter Partikelzahlen als Grenzwerte die Steigung des Anstiegs der Partikelanzahl über der Zeit genutzt wird. Vergrößert sich die Anzahl der pro Zeiteinheit auftretenden Partikel innerhalb eines Zeitintervalls dt um mehr als einen vorbestimmbaren oder vorgebbaren Faktor x, wird dieses als Grenzwertüberschreitung gewertet. Noch vorteilhafter ist es, nicht nur den Anstieg, also den Trend der Partikelanzahl heranzuziehen, sondern deren mathematische Ableitung, also die Krümmung des Trends. Eine kontinuierliche Zunahme des Anstiegs der Partikelanzahl pro Zeiteinheit über ein gewisses Zeitintervall dt, also ein Trend mit anhaltendem parabolischen oder exponentiellen Verlauf, ist ein sicherer Indikator für einen sich anbahnenden Getriebeschaden.

Hierzu kann auch die zweite Ableitung der Parükelanzahl nach der Zeit verwendet werden, Ab Erreichen eines vorgebbaren Grenzwertes der zweiten Ableitung kann ein sich sicher ergebender Getriebeschaden vorhergesagt werden.

Durch diese relative, also nicht mehr, insbesondere nur, auf absolute Partikelanzahlen basierende Auswertung ist es möglich, auch Getriebe mit unterschiedlichen technischen Eigenschaften durch ein und denselben Auswertealgorithmus zu überwachen. Beispielsweise hat sich gezeigt, dass Getriebe von unterschiedlichen Lieferanten im Normalzustand unterschiedliche Partikelanzahlen pro Zeiteinheit aufweisen, was beispielsweise auf die unterschiedliche Härte der Hohlräder der Planeteristufe zurückzuführen ist. Bei weichen Hohlrädern ist die Anzahl der Partikel auch im störungsfreien Betrieb höher als bei Getrieben mit sehr harten Hohlrädern. Eine relative Auswertung der Partikelanzahlen ermöglicht dennoch einheitliche Grenzwerte für beide Ausführungen, was die Überwachung einer Vielzahl von Windturbinen erheblich vereinfacht. Die relativen Grenzwerte werden bevorzugt zeitlich variabel, d.h. abhängig vom Betriebszustand und/oder dem Anlagenalter bzw. der Betriebsstundenzahl definiert.

Eine weitere Ausgestaltung sieht vor, dass die Auswertevorrichtung eine Speichereinheit aufweist, in der eine Vielzahl von typischen Verläufen der Partikelanzahl über der Zeit in Form von Verlaufsmustern gespeichert wird. Gleichzeitig ist jedem Verlaufsmuster auch eine oder mehrere mögliche Schadensursache(n) zugeordnet. Die Auswerteeinheit ist so gestaltet, dass sie durch Vergleich des aktuellen Verlaufs der Partikelanzahl mit den Verlaufsmustern nicht nur einen kritischen Anstieg der Partikelanzahl frühzeitig erkennt, sondern auch Rückschlüsse auf die Schadensursache ermöglicht. Somit muss bei einer erforderlichen Inspektion nicht das gesamte Getriebe auf Schäden untersucht werden, sondern lediglich einige wenige, für den spezifischen Partikelverlauf infrage kommende Bauteile. Ebenso können auch Verlaufsmuster abgelegt werden, die nicht auf Getriebeschäden zurückzuführen sind, sondern auf Fehlalarme durch Sensorfehler oder durch elektromagnetische Einstreuungen ins Messsystem (EMV-Probleme). Auf diese Weise kann die Zahl von unerwünschten Fehlalarme deutlich reduziert werden.

Die Zuordnung von Verlaufsmustern und Schadensursachen erfolgt bevorzugt durch selbstlernende Datenbanksysteme oder neuronale Netzwerke. Damit ist es möglich, die Erfahrungen mit jedem einzelnen Getriebe der Gesamtheit der zu überwachenden Windturbinen zu Nutze zu machen.

Die Datenbanksysteme sind vorzugsweise Expertensysteme. Entsprechend ist vorzugsweise vorgesehen, in einer Fernüberwachungszentrale eine relativ große Datenbank vorzusehen, die die Daten diverser Getriebe bzw. einer Vielzahl von Getrieben speichert und Ober ein selbstlernendes Expertensystem bzw. Datenbanksystem entsprechende Verlaufsmuster und Schadensursachen miteinander verknüpft. Insbesondere findet hier die Mustererkennung statt.

Verfeinert wird das erfindungsgemäße Verfahren vorteilhafterweise, wenn neben der Messung von Partikeln in der Getriebeflüssigkeit andere Getriebeparameter, insbesondere mittels Körperschallsensoren, Mikrofonen und/oder Beschleunigungssensoren, überwacht werden. Durch diese Maßnahme ist es möglich, in den Fällen, in denen eine ausschließlich automatische Überwachung mittels der Überwachung der Partikel in der Getriebeflüssigkeit keine genügende Treffersicherheit oder Betriebssicherheit gewährleistet, eine eingehendere Analyse bekannter Messwerte durch einen Experten zu Hilfe zu ziehen und somit die Treffsicherheit oder Betriebssicherheit zu erhöhen.

Weitere Parameter, die vorzugsweise berücksichtigt werden, um insbesondere natürlich auftretenden erhöhten Verschleiß von Getriebeschäden zu unterscheiden, sind Messungen von Windturbulenzen, die zu höheren Drehmomentschwankungen und damit zu höherem Verschleiß führen. Dafür werden vorzugsweise die Messwerte von Windgebern und/oder Befastungssensoren und/oder die Varianz der Leistung, der Drehzahl oder Blattwinkel erfasst und/oder ausgewertet und insbesondere zur temporären und/oder permanenten Veränderung von Grenzwerten für ein Getriebe verwendet. So wird bei stärkeren Windturbulenzen wenigstens ein Grenzwert vorzugsweise erhöht, da der unter der stärkeren Belastung auftretende höhere Verschleiß nicht zu Fehlauslösungen führen soll. Andererseits führen starke Turbulenzen häufiger zu Getriebeschäden, so dass die Erhöhung des Grenzwerts nicht beliebig mit der herrschenden Windturbulenz korreliert werden darf, sondern vorzugsweise einen absoluten maximalen Grenzwert nicht überschreiten darf.

Es ist vorteilhafterweise vorgesehen, im Falle eines Getriebeschadens wenigstens eine Schadensursache und/oder wenigstens eine beschädigte Getriebekomponente mittels, insbesondere mikroskopischer, chemischer und/oder metallurgischer, Analyse der in der Getriebeflüssigkeit enthaltenen Partikel einzugrenzen und/oder zu bestimmen. Die Unterscheidung magnetischer und nicht magnetischer Partikel ist in der kontinuierlichen Überwachung möglich. Es stehen Methoden zur. Verfügung, um Aussagen Ober die chemischen Bestandteile der Partikel zu treffen. So ist es möglich, die Partikel auf das Vorhandensein von Chrom, Nickel, Molybdän, Kupfer und anderer Bestandteile zu untersuchen, wodurch die als schadensbehaftet in Frage kommenden Bauteile eingegrenzt werden können. Auch die Messdaten konventioneller Diagnosesysteme sind flankierend einsetzbar, um ein schadhaftes Bauteil beispielsweise an Frequenzverschiebungen im Geräusch- oder Schwingungsspektrum zu identifizieren. Ein solches Kombinationssystem hat den Vorteil der sicheren und vollautomatischen Fehlererkennung.

Eine ergänzende Überwachung des Getriebes kann durch eine Drucküberwachung am Ölfilter des Getriebes erfolgen. Üblicherweise wird bei einer erfindungsgemäßen Windenergieanlage vor dem Ölfilter und hinter dem Ölfilter der Öldruck gemessen. Hierbei fällt der Öldruck durch das Filter um ca. 3 - 14 bar, abhängig von der Art des Filters, der Öltemperatur und dem Öltyp, ab. Dieser Druckabfall ändert sich in der Regel nur sehr langsam, um etwa 0.01 bar pro Tag. Eine Änderung des Druckabfalls bei vergleichbarer Öltemperatur um mehr als 0,02 bar / Tag deutet auf eine Getriebestörung (z.B. Filterverstopfung durch erhöhtes Partikelaufkommen) hin und könnte eine Statusmitteilung auslösen, bei einer Änderung um mehr als z.B. 0,05 bar könnte z.B. ein Betriebsparameter verändert werden oder die Anlage stillgesetzt werden. Eine derartige Überwachung ist natürlich nicht so sensibel wie eine direkte Partikelzählung, kann aber aufgrund des geringen Aufwands hervorragend zur redundanten Überwachung des Partikelzählers herangezogen werden.

Erfindungsgemäß ermöglicht die Partikelzählung als alleinige Überwachungsfunktion, gegebenenfalls mit flankierenden Maßnahmen, eine sichere und frühzeitige Erkennung aller Getriebeschäden. Dadurch, dass, neben der Zeit, nur eine physikalische Größe überwacht wird, ist es relativ schnell möglich, Grenzwerte zu definieren, so dass eine vollautomatische und sichere Überwachung möglich ist. Dabei ist der technische Geräteaufwand erheblich reduziert und die Überwachung durch eine Bedienperson bzw. einen Experten unnötig, so dass erhebliche Kostenvorteile einstellen. Durch die Unterscheidung von der Einlaufphase von restlicher Getriebelaufzeit ist es möglich, trotz des in der Einlaufphase unvermeidlichen höheren Aufkommens an Verschleißpartikeln in der Getriebeflüssigkeit zu erkennen, wann ein Getriebeschaden oder eine falsche Montage vorliegt. Dies ist wichtig, weil beispielsweise ein fehlerhaft montiertes Wälzlager einer Planetenstufe, das nicht erkannt wird, binnen weniger Stunden zur Zerstörung des gesamten Getriebes mit einem hohen Sachschaden führen kann.

Die Aufgabe der zugrundeliegenden Erfindung wird auch gelöst durch ein Computerprogramm mit Programmcodemitteln, die angepasst sind, ein erfindungsgemäßes Verfahren zum Überwachen eines Getriebes einer Windenergieanlage mit einem Getriebeflüssigkeitskreislauf, wie vorstehend beschrieben, auszuführen.

Die Computerimplementierung des erfindungsgemäßen Verfahrens stellt eine effektive und kostengünstige Möglichkeit zur Durchführung des Verfahrens dar, da bei modernen Windenergieanlagen die Betriebsführung bereits weitgehend computergesteuert ausgeführt wird.

Die der Erfindung zugrundeliegende Aufgabe wird ebenfalls gelöst durch ein Überwachungssystem für ein Getriebe einer Windenergieanlage mit einem Getriebeflüssigkeitskreislauf, umfassend einen Partikelzähler, eine Auswertevorrichtung und/oder eine Steuervorrichtung, wobei die Auswertevorrichtung und/oder die Steuervorrichtung als Datenverarbeitungsanlage so ausgebildet sind, dass mittels eines erfindungsgemäßen vorstehend beschriebenen Computerprogramms das ebenfalls vorstehend beschriebene erfindungsgemäße Verfahren ausführbar ist. Die Auswertevorrichtung und die Steuervorrichtung können auch in eine Datenverarbeitungsanlage bzw. einen Computer integriert sein, so dass die Funktionen der Auswertung und der Steuerung nicht in zwei getrennten Geräten realisiert sind, sondern als getrennte Funktionen in einem Gerät.

Sowohl bei dem erfindungsgemäßen Computerprogramm als auch bei dem erfindungsgemäßen Überwachungssystem ergeben sich die im. Rahmen der Beschreibung des erfindungsgemäßen Verfahrens genannten Vorteile.

Die Erfindung wird nachstehend ohne Beschränkung des allgemeinen Erfindungsgedankens anhand von Ausführungsbeispielen unter Bezugnahme auf die Zeichnungen exemplarisch beschrieben, wobei bezüglich aller im Text nicht näher erläuterten erfindungsgemäßen Einzelheiten ausdrücklich auf die schematischen Zeichnungen verwiesen wird. Es zeigen:
- Fig. 1: eine schematische Darstellung eines erfindungsgemäßen Überwachungssystems;
- Fig. 2: eine schematische Darstellung des zeitlichen Verlaufes dreier Grenzwerte gemäß der Erfindung und
- Fig. 3: eine schematische Darstellung des zeitlichen Verlaufes weiterer Grenzwerte gemäß der Erfindung.

In den folgenden Figuren sind jeweils gleiche oder gleichartige Elemente bzw. entsprechende Teile mit denselben Bezugsziffern versehen, so dass von einer entsprechenden erneuten Vorstellung abgesehen wird.

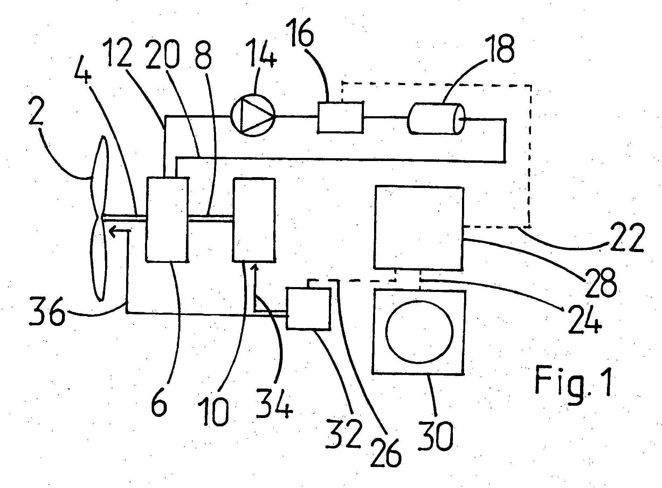

In Figur 1 ist schematisch ein erfindungsgemäßes Überwachungssystem dargestellt. Vom Triebstrang der Windenergieanlage sind die Rotorblätter 2, die Rotorwelle 4, das Getriebe 6, die Generatorwelle 8 und der Generator 10 dargestellt. Das Getriebe 6 sorgt dafür, dass die vergleichsweise langsame Umdrehung der Rotorwelle 4 in eine Rotation der Generatorwelle 8 mit hoher Umdrehungszahl umgesetzt wird.

Alternativ kann der Sensor auch vorteilhaft in einem Nebenstrom des Ölkreislaufs installiert werden. Im Nebenstrom lässt sich z.B. die Strömungsgeschwindigkeit der Partikel gegenüber dem Hauptstrom deutlich reduzieren, wodurch eine genauere Analyse der Partikel, z.B. bzgl, der Partikelgröße oder der Zusammensetzung durchgeführt werden kann. Natürlich sind die Grenzwerte für eine Auswertung im Nebenstrom an die Gegebenheiten anzupassen, d.h. in der Regel erheblich zu reduzieren.

Das Getriebe 6 ist Teil eines Getriebeflüssigkeitskreislaufs, das neben dem Getriebe 6 eine Leitung 12 für Getriebeöl, eine Ölpumpe 14, einen Ölfilter 18 und eine Rückleitung 20 für das Getriebeöl umfasst. Ein Ölzwischenbehälter oder eine Ölwanne sind nicht dargestellt, können aber vorhanden sein. Zwischen der Ölpumpe 14 und dem Ölfilter 18, in dem Verschleißpartikel aus dem Getriebe 6 aus dem Kreislauf herausgefiltert werden, ist ein Partikelzähler 16 angeordnet, in dem Partikel im Getriebeflüssigkeitskreislauf gezählt werden, bevor sie aus dem Getriebeflüssigkeitskreislauf mittels des Ölfilters 18 herausgefiltert werden.

Der Partikelzähler 16 ist insbesondere ein induktiver Partikelzähler, mittels dessen metallische bzw. magnetisierbare Partikel gemessen werden. Es kann auch ein optischer Partikelzähler oder eine Kombination eines oder mehrerer optischer und induktiver Partikelzähler vorgesehen sein. Die Messdaten des Partikelzählers, die entweder ein digitales oder ein analoges Signal sein können, werden über eine gestrichelt dargestellte Datenleitung 22 an eine Auswertevorrichtung 28 weitergeleitet. In der Auswertevorrichtung 28 werden die kumulative Anzahl von Partikeln, die Anzahl von Partikeln pro Zeiteinheit oder deren Veränderung, die Größe der Partikel, die Größenverteilung der Partikel, die Änderung der Größenverteilung der Partikel, die Arten der Partikel, die Verteilung der Partikelarten und/oder deren zeitliche Veränderung ausgewertet und auf das Vorliegen von Getriebeschäden bzw. auf den allgemeinen Getriebezustand hin analysiert. Dazu wird der Messwert oder werden die Messwerte mit in der Analysevorrichtung 28 gespeicherten Grenzwerten verglichen.

Als Ergebnis der Überwachung ist es möglich, dass die Auswertevorrichtung 28 über eine gestrichelt dargestellte Datenleitung 24 eine Statusmitteilung an eine Anzeigevorrichtung 30 sendet. Die Statusmitteilung kann eine Mitteilung über den momentanen Getriebezustand, eine Alarmmitteilung, eine Nachricht über eine Änderung eines Betriebsparameters der Windenergieanlage oder eine Mitteilung sein, dass eine instandhaltungsmaßnahme angesetzt wird.

Die Anzeigevorrichtung 30 ist beispielsweise in einer Fernüberwachungszentrale für einen Windpark mit mehreren Windenergieanlagen angeordnet. Bei der Anzeigevorrichtung 30 kann es sich auch um einen Computermonitor handeln, der am Ort der Auswertevorrichtung 28 mit der Auswertevorrichtung 28 verbunden ist, wobei die Auswertevorrichtung 28 als Computer ausgebildet ist. Die Auswertevorrichtung 28 kann beispielsweise in der Gondel einer Windenergieanlage oder in einer Fernüberwachungszentrale angeordnet sein. Es kann auch sowohl in der Gondel einer Windenergieanlage als auch in einer Fernüberwachungszentrale eine Anzeigevorrichtung 30 vorgesehen sein.

Über eine gestrichelt dargestellte Datenleitung 26 steuert die Auswertevorrichtung 28 eine Steuervorrichtung 32 an, die, abhängig von der Art des Signals, verschiedene Betriebsparameter verändern kann. Es ist in Fig. 1 dargestellt, dass die Steuervorrichtung 32 Ober ein Steuersignal 34 am Generator 10 das Generatormoment verändert. So kann das Generatormoment in Extremfällen auf Null gesetzt werden. Alternativ oder gleichzeitig damit ist es möglich, dass die Steuervorrichtung 32 über ein Steuersignal 36 den Rotorblatteinstellwinkel steuert. Auch über den Rotorblatteinstellwinkel kann die Belastung des Getriebes 6 vermindert werden. Der Rotorblatteinstellwinkel kann soweit verändert werden, dass in Fahnenstellung der Rotor sich nicht mehr dreht und die Windenergieanlage still gesetzt wird.

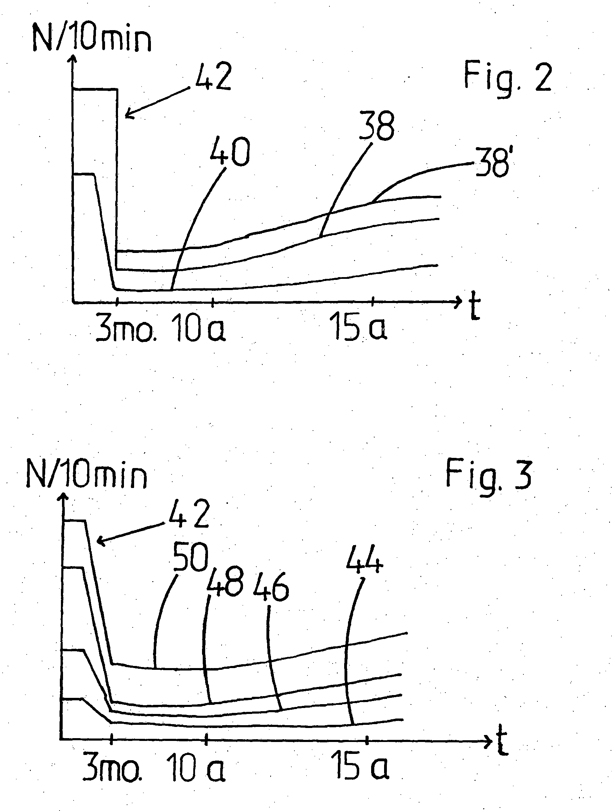

Figur 2 zeigt eine schematische Darstellung der zeitlichen Verläufe dreier zeitabhängiger Grenzwerte 38, 38', 40. Dabei ist ein höherer Grenzwert 38 für weniger kritische kleinere Partikel vorgesehen und ein niedrigerer Grenzwert 40 für sicherheitsrelevante größere Partikel, die auf einen schwerwiegenden Getriebeschaden hinweisen. Es ist außerdem ein zweiter Grenzwert 38' für kleinere Partikel dargestellt, wobei der Unterschied zwischen den Grenzwerten 38 und 38' darin besteht, dass bei Erreichen des errechneten Messwertes des Grenzwertes 38 eine Statusmitteilung erfolgt, während bei Erreichen des zweiten Grenzwertes 38' eine Betriebsparameterveränderung erfolgt, mit der das Getriebe entlastet wird.

Auf der horizontalen Achse ist mit "t" der Zeitpfeil bezeichnet, wobei auf der horizontalen Achse drei verschiedene Zeitpunkte eingezeichnet sind, nämlich drei Monate (mit Abkürzung "mo."), ein Jahr und drei Jahre, wobei die Abkürzung "a" für "annus" = "Jahr" benutzt wird. Auf der vertikalen Achse ist als Einheit die Anzahl von Partikeln in einem Zeitraum von 10 Minuten mit willkürlichen Einheiten bezeichnet.

Der erste zeitliche Abschnitt bis drei Monate entspricht einer Einlaufphase 42 des Getriebes, in dem sich die einzelnen Komponenten des Getriebes aufeinander einschleifen. In diesen Zeitraum fällt eine große Zahl von Partikeln an, die allerdings keinen erhöhten Verschleiß bedeuten, sondern in der Einiaufphase 42 normal und akzeptabel sind. Ein niedriger Grenzwert in der Einlaufphase 42 würde zu unnötig hohen Stillstandszeiten führen, obwohl kein erhöhter Verschleiß und keine Getriebeschäden Vorliegen. Die Einlaufphase 42, die hier beispielhaft auf drei Monate terminiert ist, kann, abhängig vom Getriebetyp und Windenergieanlagentyp sowie der anfänglichen Betriebsdauer, länger oder kürzer sein. Die Einlaufphase 42 kann auch durch Messung stark abnehmender Partikelzahlen in der Getriebeflüssigkeit für ein Getriebe individuell abgegrenzt werden.

Am Ende der Einlaufphase 42 sind zwei verschiedene Szenarien des zeitlichen Verlaufs der Grenzwerte dargestellt. Bei dem höheren Grenzwert 38 ist am Ende der Einlaufphase 42 dargestellt, dass nach einem konstanten Wert des Grenzwertes 38 bzw. 38' abrupt ein deutlich niedrigerer Grenzwert eingenommen wird, der dem normalen Betrieb des Getriebes in der Windenergieanlage nach der Einlaufphase 42 entspricht. Eine Alternative hierzu ist im Verlauf des Grenzwertes 40 gezeigt. Nach einem sehr hohen Anfangswert wird der Abnahme des Verschleißes gegen Ende der Einlaufphase 42 Rechnung getragen, indem der Grenzwert schon vor Ende der Einlaufphase 42 vom anfänglichen maximalen Wert allmählich auf den Betriebswert nach der Einlaufphase 42 heruntergeführt wird.

In der auf die Einlaufphase 42 folgenden Zeit, im Beispiel gemäß Figur 2 zwischen drei Monaten und 10 Jahren, die aber auch kürzer oder länger sein kann, sind die Grenzwerte 38, 38', 40 weitgehend stabil. Die Getriebebauteile zeigen noch keine Ermüdungserscheinungen, so dass Grenzwerte niedrig angesetzt werden. Verstärkter Verschleiß in dieser Phase deutet auf Probleme und Schäden im Getriebe hin.

Nach dieser stabilen Phase ist es bekannt, dass die Materialien der Getriebekomponenten langsam ermüden, wodurch vermehrt Verschleiß und eine höhere Anzahl von Partikeln in der Getriebeflüssigkeit auftreten, ohne dass deswegen die Sicherheit des Betriebes beeinträchtigt wäre. Daher steigen die Grenzwerte 38, 38', 40 in diesem Zeitraum leicht an. Damit wird vermieden, dass das steigende "Rauschen" der Messwerte aufgrund der Alterungserscheinungen unnötige Alarme oder Stillstandszeiten verursacht.

Figur 3 zeigt eine schematische Darstellung des zeitlichen Verlaufes von Grenzwerten 44, 46, 48, 50 über die Lebensdauer eines Getriebes, wobei der Grenzwert 44 für den Stillstand, der Grenzwert 46 für den Trudelbetrieb, der Grenzwert 48 den Teillastbetrieb und der Grenzwert 50 für den Volllastbetrieb der Windenergieanlage bzw. des Getriebes gelten. In diesen vier Betriebsarten bzw. Betriebszyklen wird das Getriebe unterschiedlich belastet, was sich in unterschiedlichen Verschleiß und Partikelzahlen und folgend daraus unterschiedlichen Grenzwerten 44 bis 50 niederschlägt. Ansonsten entsprechen die Achsen in Figur denen aus Figur 2.

Für andere mögliche Messwerte würden die zeitlichen Darstellungen der Grenzwerte ähnlich aussehen wie die in Figur 2 und Figur 3 dargestellten.

In mathematisch reziproker Weise kann statt eines zeitlich veränderlichen Grenzwertes auch ein konstanter Grenzwert verwendet werden, an dem ein nach Art des Messwertes und nach Betriebszyklus, also Betriebsdauer und Betriebsart, gewichteter Messwert verglichen wird. Entsprechend wird bei dieser Vorgehensweise während der Einlaufphase 42 ein sehr kleiner Gewichtungsfaktor genommen, was umgekehrt einem großen Grenzwert entspricht, während der Gewichtungsfaktor nach der Einlaufphase groß wird und im Laufe des Lebens eines Getriebes langsam abnimmt, wodurch der in Figur 2 bzw. Figur 3 dargestellte langsame Anstieg des Grenzwertes nachgebildet wird.

Ebenso können verschiedene Messdatentypen gewichtet oder ungewichtet miteinander verknüpft werden, um beispielsweise aus der Verteilung der Arten von Partikeln in der Getriebeflüssigkeit und der Verteilung der Partikeigrößen und deren zeitlichen Änderungen ebenso wie auf der Anzahl der Partikel aussagekräftige Parameter über den Getriebezustand bzw, das Vorliegen bestimmter Getriebeschäden zu ziehen. So kann in der statistischen Auswertung der Messdaten eine Vielzahl von Getrieben von Windenergieanlagen gefunden werden, dass bestimmte Getriebeschäden bestimmte Signaturen in den Messwerten aufweisen und bei Auftritt einer solchen Signatur ein entsprechender Getriebeschaden angezeigt und gegebenenfalls die entsprechenden Gegenmaßnahmen eingeleitet werden.

Mittels des erfindungsgemäßen Verfahrens und der erfindungsgemäßen Software und des erfindungsgemäßen Systems ist es möglich, eine flexible und vielstufige überwachungsfunktion zu realisieren, mittels der abgestuft auf das Auftreten erhöhten Verschleißes reagiert werden kann, beispielsweise durch eine Aufeinanderfolge von Statusmitteilungen, Verringerung der auf das Getriebe einwirkenden Leistungen und im extremen Fall Stillsetzen der Windenergieanlage.

### Beaucszeichenliste

- 2: Rotorblätter
- 4: Rotorwelle
- 6: Getriebe
- 8: Generatorwelle
- 10: Generator
- 12: Leitung für Getriebeöl
- 14: Ölpumpe
- 16: Partikelzähler
- 18: Ölfilter
- 20: Rückleitung für Getriebeöl
- 22, 24, 26: Datenleitung
- 28: Auswertevorrichtung
- 30: Anzeigevorrichtung
- 32: Steuervorrichtung
- 34: Steuersignal an den Generator
- 36: Steuersignal für den Rotorblatteinstellwinkel
- 38, 38': zeitabhängiger Grenzwert für kleine Partikel,
- 40: zeitabhängiger Grenzwert für größere Partikel
- 42: Einlaufphase
- 44: zeitabhängiger Grenzwert bei Stillstand
- 46: zeitabhängiger Grenzwert bei Trudelbetrieb
- 48: zeitabhängiger Grenzwert bei Teillast
- 50: zeitabhängiger Grenzwert bei Volllast

## Patentansprüche

1. Verfahren zum Überwachen eines Getriebes (6) einer Windenergieanlage mit einem Getriebeflüssigkeitskreislauf (6, 12, 14, 16, 18, 20), bei dem mittels wenigstens eines Partikelfählers (16) die Anzahl und/oder die Größe und/oder die Art von in der Getriebeflüssigkeit enthaltenen und durch einen GetriebeflüssigKeitsleitungsquerschnitt strömenden Partikeln durch Messung bestimmt wird **dadurch gekennzeichnet, dass** bei Überschreiten wenigstens eines ersten Grenzwertes (38, 40, 44, 46, 48, 50) durch einen Messwert eine Statusmitteilung erzeugt wird und bei Überschreiten wenigstens eines zweiten Grenzwertes (38, 40, 44, 46, 48, 50) durch einen Messwert ein Betriebsparameter (34, 36) der Windenergieanlage verändert wird.

2. Verfahren nach Anspruch 1, **dadurch gekennzeichnet, dass** metallische Partikel mittels wenigstens eines induktiven Partikelzählers (16) gemessen werden, wobei insbesondere die Messung im Getriebeflüssigkeitskreislauf (6, 12, 14, 16, 18, 20) stromaufwärts eines Partikelfilters (18) stattfindet, und wobei der veränderte Betriebsparameter (34, 36) ein Blatteinstellwinkel (36) und/oder ein Generatormoment (34) ist und/oder dass die Windenergieanlage stillgesetzt wird.

3. Verfahren nach Anspruch 1 oder 2, **dadurch gekennzeichnet, dass** wenigstens ein Grenzwert (38, 40, 44, 46, 48, 50) eine kumulierte Anzahl von Partikeln, eine Anzahl von Partikeln pro Zeiteinheit, eine Veränderung der Anzahl von Partikeln pro Zeiteinheit, eine absolute Größe von Partikeln, eine Größenverteilung von Partikeln, eine Veränderung der Partikelgrößenverteilung, die Art der Partikel oder eine Verteilung verschiedener Partikelarten oder eine Veränderung der Verteilung von Partikelarten oder ein aus verschiedenen Messwerten gebildeter abgeleiteter Wert ist.

4. Verfahren nach Anspruch 3, **dadurch gekennzeichnet, dass** der Grenzwert (38, 40, 44, 46, 48, 50) eine Anzahl von 25 bis 50 Partikeln in 10 Minuten, eine Anzahl von 500 bis 1.000 Partikeln in 24 Stunden oder eine kumulierte Anzahl von 300.000 bis 400.000 Partikeln ist.

5. Verfahren nach einem der Ansprüche 1 bis 4, **dadurch gekennzeichnet, dass** wenigstens ein Grenzwert (38, 40, 44, 46, 48, 50) abhängig vom Betriebszustand der Windenergieanlage verändert wird, insbesondere Stillstand, Trudel-, Teillast- und/oder Volllastbetrieb des Getriebes (6) und/oder abhängig vom Lebens- oder Verschleißalter des Getriebes (6), der Betriebsstunden und/oder der Volllaststunden des Getriebes (6), wobei insbesondere Grenzwerte (38, 40, 44, 46, 48, 50) für wenigstens einen Messwert von Stillstand über Trudelbetrieb, Teillastbetrieb bis hin zu Volllastbetrieb schrittweise erhöht werden.

6. Verfahren nach einem der Ansprüche 1 bis 5, **dadurch gekennzeichnet, dass** wenigstens ein Grenzwert (38, 40, 44, 46, 48, 50) während einer Einlaufphase (42) höher gesetzt wird als nach der Einlaufphase (42), wobei insbesondere nach der Einlaufphase der wenigstens eine nach der Einlaufphase (42) herabgesetzte Grenzwert (38, 40, 44, 46, 48, 50) mit zunehmender Laufzeit des Getriebes (6) erhöht wird.

7. Verfahren nach einem der Ansprüche 1 bis 6, **dadurch gekennzeichnet, dass** wenigstens ein Grenzwert (38, 40, 44, 46, 48, 50) für einen Messwert und/oder der zeitliche Verlauf des wenigstens einen Grenzwerts (38, 40, 44, 46, 48, 50) durch eine statistische Auswertung von Messwertdaten mehrerer Windenergieanlagen mit gleichen oder ähnlichen Getriebetypen in Abhängigkeit vom Betriebszyklus bestimmt wird, wobei die Streuung der Messwerte bei der Bestimmung des Grenzwerts (38, 40, 44, 46, 48, 50) einfließt, wobei der wenigstens eine in der statistischen Auswertung bestimmte Grenzwert (38, 40, 44, 46, 48, 50) einer mehr als 95%igen, insbesondere einer 99%igen, Fehlererkennungsquote entspricht.

8. Verfahren nach Anspruch 7, **dadurch gekennzeichnet, dass** für ein Getriebe (6) wenigstens ein Grenzwert (38, 40, 44, 46, 48, 50) anhand der Messwerte und/oder des bisherigen zeitlichen Verlaufs der Messwerte des Getriebes (6) korrigiert wird, wobei insbesondere Getriebe, bei denen Getriebeschäden aufgetreten sind, bei der statistischen Auswertung zur Ermittlung eines Grenzwerts (38, 40, 44, 46, 48, 50) oder des zeitlichen Verlaufs eines Grenzwerts (38, 40, 44, 46, 48, 50), der einem sicheren Betrieb des Getriebes (6) entspricht, nicht berücksichtigt werden.

9. Verfahren nach Anspruch 8, **dadurch gekennzeichnet, dass** Getriebe, bei denen Getriebeschäden aufgetreten sind, zur Erkennung von Getriebeschäden berücksichtigt werden, insbesondere anhand der für bestimmte Getriebeschäden charakteristischen zeitlichen Verläufe eines oder mehrerer Messwerte.

10. Verfahren nach einem der Ansprüche 1 bis 9, **dadurch gekennzeichnet, dass** aus der Analyse des gemessenen zeitlichen Verlaufs wenigstens eines Messwerts, insbesondere durch Vergleich des Messwerts mit Messwerten von anderen Windenergieanlagen mit gleichen oder ähnlichen Getriebetypen, ein Schadensfall erkannt wird und eine entsprechende Statusmitteilung generiert wird und/oder Betriebsparameter (34, 36) der Windenergieanlage geändert werden.

11. Verfahren nach einem der Ansprüche 1 bis 10, **dadurch gekennzeichnet, dass** aus der Analyse des gemessenen zeitlichen Verlaufs wenigstens eines Messwerts, insbesondere durch Vergleich des Messwerts mit Messwerten von anderen Windenergieanlagen mit gleichen oder ähnlichen Getriebetypen, die zu erwartende Restlaufzeit des Getriebes (6) und/oder ein Termin für eine Instandhaltungsmaßnahme für das Getriebe (6) ermittelt wird, wobei insbesondere mehrere Messwerte mit verschiedenen Gewichtungen, insbesondere für verschiedene Partikelgrößen, ausgewertet werden, und wobei die Gewichtungen der Messwerte ihrer Indikatoreigenschaft für Alarmzustände und/oder Schadenszustände eines Getriebes (6) entsprechen.

12. Verfahren nach einem der Ansprüche 1 bis 11, **dadurch gekennzeichnet, dass** neben der Messung von Partikeln in der Getriebeflüssigkeit andere Getriebeparameter, insbesondere mittels Körperschallsensoren, Mikrophonen und/oder Beschleunigungssensoren, überwacht werden, wobei insbesondere außerdem die Messwerte von Windgebern und/oder Belastungssensoren und/oder die Varianz der Leistung, der Drehzahl oder Blattwinkel erfasst und/oder ausgewertet werden und zur temporären und/oder permanenten Veränderung von Grenzwerten (38, 40, 44, 46, 48, 50) für ein Getriebe (6) verwendet werden.

13. Verfahren nach einem der Ansprüche 1 bis 12, **dadurch gekennzeichnet, dass** im Falle eines Getriebeschadens wenigstens eine Schadensursache und/oder wenigstens eine beschädigte Getriebekomponente mittels, insbesondere mikroskopischer, chemischer und/oder metallurgischer, Analyse der in der Getriebeflüssigkeit enthaltenen Partikel eingegrenzt und/oder bestimmt wird.

14. Computerprogramm mit Programmcodemitteln, die angepasst sind, um ein Verfahren zum Überwachen eines Getriebes (6) einer Windenergieanlage mit einem Getriebeflüssigkeitskreislauf nach einem der Ansprüche 1 bis 13 auszuführen.

15. Überwachungssystem für ein Getriebe (6) einer Windenergieanlage mit einem Getriebeflüssigkeitskreislauf, umfassend einen Partikelzähler (16), eine Auswertevorrichtung (28) und/oder eine Steuervorrichtung (32), wobei die Auswertevorrichtung (28) und/oder die Steuervorrichtung (32) als Datenverarbeitungsanlage so ausgebildet sind, dass mittels eines Computerprogramms nach Anspruch 14 ein Verfahren nach einem der Ansprüche 1 bis 13 ausführbar ist.

## Claims

1. A method of monitoring a transmission system (6) of a wind turbine with a transmission system fluid circuit (6, 12, 14, 16, 18, 20), in which, by means of at least one particle counter (16), the number and/or the size and/or the type of particles contained in the transmission system fluid and flowing through a transmission system fluid conduit cross-section is determined by measurement, **characterised in that** when at least a first threshold value (38, 40, 44, 46, 48, 50) is exceeded by a measured value, a status notification is generated and when at least one second threshold value (38, 40, 44, 46, 48, 50) is exceeded by a measured value an operating parameter (34, 36) of the wind turbine is changed.

2. A method as claimed in Claim 1, **characterised in that** metallic particles are measured by at least one inductive particle counter (16), wherein, in particular, the measurement in the transmission system fluid circuit (6, 12, 14, 16, 18, 20) occurs upstream of a particle filter (18) and wherein the changed operating parameter (34, 36) is a blade pitch angle (36) and/or a generator torque (34) and/or the wind turbine is stopped.

3. A method as claimed in Claim 1 or 2, **characterised in that** at least one threshold value (38, 40, 44, 46, 48, 50) is a cumulative number of particles, a number of particles per unit of time, a change in the number of particles per unit of time, an absolute size of particles, a size distribution of particles, a change in the particle size distribution, the type of the particles or a distribution of different particles or a change in the distribution of types of particle or a value derived from different measured values.

4. A method as claimed in Claim 3, **characterised in that** the threshold value (38, 40, 44, 46, 48, 50) is a number of 25 to 50 particles in 10 minutes, a number of 500 to 1,000 particles in 24 hours or a cumulative number of 300,000 to 400,000 particles.

5. A method as claimed in one of Claims 1 to 4, **characterised in that** at least one threshold value (38, 40, 44, 46, 48, 50) is changed in dependence on the operational state of the wind turbine, particularly standstill, idling operation, partial load operation and/or full load operation of the transmission system (6) and/or in dependence on the service age or wear age of the transmission system, the operational hours and/or the full load hours of the transmission system (6), wherein, in particular, threshold values (38, 40, 44, 46, 48, 50) for at least one measured value from standstill via idling operation, partial load operation up to full load operation are increased incrementally.

6. A method as claimed in one of Claims 1 to 5, **characterised in that** at least one threshold value (38, 40, 44, 46, 48, 50) is set higher during a run-in period (42) than after the run-in period (42), wherein, in particular, after the run-in period the at least one threshold value (38, 40, 44, 46, 48, 50), which is reduced after the run-in period (42), is increased with increasing run time of the transmission system.

7. A method as claimed in one of Claims 1 to 6, **characterised in that** at least one threshold value (38, 40, 44, 46, 48, 50) for a measured value and/or the trend over time of the at least one threshold value (38, 40, 44, 46, 48, 50) is determined by a statistical analysis of measured value data from a plurality of wind turbines with the same or similar types of transmission system in dependence on the operational cycle, wherein the variance of the measured values has an influence on the determination of the threshold value (38, 40, 44, 46, 48, 50), wherein the at least one threshold value (38, 40, 44, 46, 48, 50) determined in the statistical analysis corresponds to a more than 95%, particularly a 99%, fault detection rate.

8. A method as claimed in Claim 7, **characterised in that** for a transmission system (6), at least one threshold value (38, 40, 44, 46, 48, 50) is corrected on the basis of the measured values and/or the previous pattern over time of the measured values from the transmission system (6), wherein, particularly transmission systems in which operational defects have occurred, are not taken account of in the statistical analysis to determine a threshold value (38, 40, 44, 46, 48, 50) or the pattern over time of a threshold value (38, 40, 44, 46, 48, 50), which corresponds to reliable operation of the transmission system (6).

9. A method as claimed in Claim 8, **characterised in that** transmission systems, in which operational faults have occurred, are taken account of to detect operational faults, particularly with the aid of the patterns over time characteristic of certain operational faults of one or more measured values.

10. A method as claimed in one of Claims 1 to 9, **characterised in that** from the analysis of the measured pattern over time of at least one measured value, particularly by comparison of the measured value with measured values from other wind turbines with the same or similar types of transmission system, a damage event is detected and a corresponding status notification is generated and/or operational parameters (34, 36) of the wind turbine are altered.

11. A method as claimed in one of Claims 1 to 10, **characterised in that** from the analysis of the measured pattern over time of at least one measured value, particularly by comparison of the measured value with measured values from other wind turbines with the same or similar types of transmission system, the remaining life to be expected of the transmission system and/or a date for a maintenance measure for the transmission system (6) is determined, wherein, in particular, a plurality of measured values with different weightings, particularly for different particle sizes, are analysed and wherein the weightings of the measured values correspond to their indicative properties for alarm conditions and/or fault conditions of a transmission system (6).

12. A method as claimed in one of Claims 1 to 11, **characterised in that** in addition to the measurement of particles in the transmission system fluid, other transmission system parameters are monitored, particularly by means of structural noise sensors, microphones and/or acceleration sensors, wherein, in particular, the measured values from wind sensors and/or load sensors and/or the variance in the output, the speed or blade pitch angle are also detected and/or analysed and are used for the temporary and/or permanent change of threshold values (38, 40, 44, 46, 48, 50) for a transmission system (6).

13. A method as claimed in one of Claims 1 to 12, **characterised in that** in the event of operational damage, at least one cause of damage and/or at least one damaged transmission system component is localised and/or determined by means of, in particular, microscopic, chemical and/or metallurgical analysis of the particles contained in the transmission system fluid.

14. A computer program with program code means, which are adapted to perform a method for monitoring a transmission system (6) of a wind turbine with a transmission system fluid circuit as claimed in one of Claims 1 to 13.

15. A monitoring system for a transmission system (6) for a wind turbine with a transmission system fluid circuit including a particle counter (16), an analysis device (28) and/or a control device (32), wherein the analysis device (26) and/or the control device (32) is constructed in the form of a data processing device so that a method as claimed in one of Claims 1 to 13 can be performed by means of a computer program as claimed in Claim 14.

## Revendications

1. Procédé de surveillance d'une transmission (6) d'une éolienne comprenant un circuit hydraulique de transmission (6, 12, 14, 16, 18, 20), dans lequel le nombre et / ou taille et / ou le type de particules contenues dans le fluide de transmission, circulant dans du fluide présent dans une portion d'un conduit de la transmission, est déterminé par mesure au moyen d'au moins un compteur de particules (16), **caractérisé en ce que**, lors du dépassement d'au moins une première valeur limite (38, 40, 44, 46, 48, 50) par une valeur mesurée, un message d'état est généré, et **en ce que**, lors du dépassement d'au moins une deuxième valeur limite (38, 40, 44, 46, 48, 50) par une valeur mesurée, un paramètre de fonctionnement (34, 36) de l'éolienne est modifié.

2. Procédé selon la revendication 1, **caractérisé en ce que** des particules métalliques sont mesurées par au moins un compteur de particules (16) à induction, dans lequel, en particulier, la mesure dans le circuit hydraulique de la transmission (6, 12, 14, 16, 18, 20) a lieu en amont d'un filtre à particules (18) et dans lequel le paramètre de fonctionnement modifié (34, 36) correspond à un angle de pas de pale (36) et / ou à un couple de générateur (34) et / ou à l'arrêt de l'éolienne.

3. Procédé selon la revendication 1 ou la revendication 2, **caractérisé en ce qu'**au moins une valeur limite (38, 40, 44, 46, 48, 50) est dérivée à partir d'un nombre cumulé de particules, d'un nombre de particules par unité de temps, d'un changement du nombre de particules par unité de temps, d'une taille absolue de particules, d'une granulométrie des particules, d'un changement dans la granulométrie des particules, du type de particules ou de la distribution de différents types de particules ou d'une modification de la distribution des différents types de particules, ou à partir de diverses valeurs mesurées.

4. Procédé selon la revendication 3, **caractérisé en ce que** la valeur limite (38, 40, 44, 46, 48, 50) est un nombre compris entre 25 et 50 particules en 10 minutes, un nombre compris entre 500 et 1000 particules en 24 heures ou un nombre cumulé compris entre 300 000 et 400 000 particules.

5. Procédé selon l'une quelconque des revendications 1 à 4, **caractérisé en ce qu'**au moins une valeur limite (38, 40, 44, 46, 48,50) est modifiée en fonction de l'état de fonctionnement de l'éolienne, en particulier de l'état d'arrêt, du fonctionnement en drapeau, du fonctionnement à charge partielle et / ou du fonctionnement à pleine charge de la transmission (6) et / ou en fonction de la durée de service ou de l'usure de la transmission (6), des heures de fonctionnement et / ou des heures de fonctionnement de la transmission (6) à pleine charge, en particulier en fonction des valeurs limites (38, 40, 44, 46, 48, 50) pour au moins une valeur mesurée depuis l'arrêt du fonctionnement en drapeau, le fonctionnement à charge partielle étant augmenté progressivement en fonctionnement à pleine charge.

6. Procédé selon l'une quelconque des revendications 1 à 5, **caractérisé en ce qu'**au moins une valeur limite (38, 40, 44, 46, 48, 50) est plus élevée durant une phase de rodage (42) qu'après la phase de rodage (42), dans lequel, en particulier, après la phase de rodage (42) avec au moins une valeur limite (38, 40, 44, 46, 48, 50) réduite, la valeur limite est augmentée au fur et à mesure de la durée de service de la transmission (6).

7. Procédé selon l'une quelconque des revendications 1 à 6, **caractérisé en ce qu'**au moins une valeur limite (38, 40, 44, 46, 48, 50) pour la mesure et / ou l'évolution dans le temps d'au moins une valeur limite (38, 40, 44 , 46, 48, 50) est déterminée par une analyse statistique des données mesurée sur plusieurs éoliennes comprenant les mêmes types de transmissions ou des transmissions similaires en fonction du cycle de fonctionnement, la dispersion des valeurs de mesure pour déterminer la valeur limite (38, 40, 44, 46, 48, 50) étant inscrite, et, lors de l'évaluation statistique d'au moins une valeur limite (38, 40, 44, 46, 48, 50), le taux de détection d'erreurs correspondant à plus de 95%, en particulier à 99%.

8. Procédé selon la revendication 7, **caractérisé en ce que**, pour une transmission (6), au moins une valeur limite (38, 40, 44, 46, 48, 50) est corrigée en utilisant les valeurs mesurées et / ou l'évolution dans le temps des valeurs de la transmission (6) mesurées précédemment, dans lequel, en particulier, une transmission dans laquelle des dommages se sont produits et ne sont pas pris en considération dans l'analyse statistique pour déterminer une valeur limite (38, 40, 44, 46, 48, 50) ou une valeur limite évoluant dans le temps (38, 40, 44, 46, 48, 50), correspondant à un fonctionnement de la transmission (6) en toute sécurité.

9. Procédé selon la revendication 8, **caractérisé en ce que** des dommages dans la transmission, lorsqu'ils se sont produits, et sont pris en compte pour la détection de dommages dans la transmission le temps d'une ou de plusieurs mesures, en particulier sur la base de dommages caractéristiques dans une transmission.

10. Procédé selon l'une quelconque des revendications 1 à 9, **caractérisé en ce qu'**à partir de l'analyse de l'évolution dans le temps d'au moins une valeur mesurée, en particulier par comparaison de la valeur mesurée avec des valeurs mesurées dans d'autres éoliennes comprenant les mêmes types de transmissions ou des transmissions similaires, un endommagement est détecté et un message d'état correspondant est généré et / ou des paramètres de fonctionnement (34, 36) de l'éolienne sont modifiés.

11. Procédé selon l'une quelconque des revendications 1 à 10, **caractérisé en ce qu'**à partir de l'analyse de l'évolution dans le temps d'au moins une valeur mesurée, en particulier par comparaison de la valeur mesurée avec des valeurs mesurées d'autres éoliennes comprenant les mêmes types de transmissions ou des transmissions similaires, la durée de service restante prévue pour la transmission (6) et / ou la date d'une maintenance de la transmission (6) est déterminée, dans lequel, en particulier, une pluralité de valeurs de mesure sont faites mesurant des poids différents, et en particulier des particules ayant des tailles différentes, et dans lequel des pondérations des valeurs, mesurées par leur indicateur de propriété, correspondent pour des conditions d'alarme et / ou des états de dommage d'une transmission (6).

12. Procédé selon l'une quelconque des revendications 1 à 11, **caractérisé en ce qu'**en plus de la mesure de particules dans le fluide de transmission, d'autre paramètres de transmission sont surveillés, en particulier au moyen de capteurs d'émission acoustique, de microphones et / ou d'accéléromètres, dans lequel en particulier, les valeurs mesurées par un anémomètre et / ou par des capteurs de force et / ou la variation de la puissance, la vitesse de rotation ou l'angle d'incidence des pales sont enregistrés et / ou sont évalués et peuvent être utilisés pour le changement temporaire et / ou permanent des valeurs limites pour la transmission (6).

13. Procédé selon l'une quelconque des revendications 1 à 12, **caractérisé en ce que** dans le cas d'un dommage à la transmission, au moins une cause du dommage et / ou au moins un composant de la transmission endommagée est isolé et / ou déterminé en particulier au moyen d'une analyse microscopique, chimique et / ou métallurgique des particules contenues dans le liquide de transmission.

14. Programme informatique comprenant des outils formant des moyens de codes de programmation adaptés pour exécuter un procédé de surveillance d'une transmission (6) d'une éolienne comprenant un circuit hydraulique de transmission selon l'une quelconque des revendications 1 à 13.

15. Système de surveillance pour la transmission (6) d'une éolienne comprenant un circuit hydraulique de transmission, un compteur de particules (16), un dispositif d'évaluation (28) et / ou un dispositif de commande (32), dans lequel le dispositif d'évaluation (28) et / ou le dispositif de commande (32) sont configurés en tant que système de traitement de données de telle sorte que le procédé selon l'une quelconque des revendications 1 à 13 est exécutable par l'intermédiaire d'un programme informatique selon la revendication 14,.
